(19) Europäisches Patentamt
European Patent Office
Office européen des brevets

(11) EP 3 189 838 A1

(12) **EUROPEAN PATENT APPLICATION**
published in accordance with Art. 153(4) EPC

(43) Date of publication:
**12.07.2017 Bulletin 2017/28**

(21) Application number: **15837719.2**

(22) Date of filing: **01.09.2015**

(51) Int Cl.:
***A61K 31/437*** (2006.01) ***A61K 31/4365*** (2006.01)
***A61K 31/4465*** (2006.01) ***A61K 31/4709*** (2006.01)
***A61K 31/519*** (2006.01) ***A61K 31/616*** (2006.01)
***A61K 45/00*** (2006.01) ***A61P 7/02*** (2006.01)

(86) International application number:
**PCT/JP2015/074816**

(87) International publication number:
**WO 2016/035780 (10.03.2016 Gazette 2016/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **02.09.2014 JP 2014177619**

(71) Applicant: **Daiichi Sankyo Company, Limited**
**Tokyo 103-8426 (JP)**

(72) Inventors:
- **MORISHIMA, Yoshiyuki**
  **Tokyo 140-8710 (JP)**
- **HONDA, Yuko**
  **Tokyo 140-8710 (JP)**
- **KAMISATO, Chikako**
  **Tokyo 140-8710 (JP)**

(74) Representative: **Fairbairn, Angus Chisholm**
**Marks & Clerk LLP**
**90 Long Acre**
**London WC2E 9RA (GB)**

(54) **PHARMACEUTICAL COMPOSITION FOR INHIBITING FORMATION OF THROMBI AND EMBOLI AFTER PLACEMENT OF STENT**

(57) It is intended to provide a novel pharmaceutical composition that can suppress thromboembolism after stent placement. The present invention provides a pharmaceutical composition for the suppression of thromboembolism after stent placement, comprising edoxaban or a pharmaceutically acceptable salt thereof, or a hydrate of the compound or the salt.

EP 3 189 838 A1

## Description

### Technical Field

The present invention relates to a pharmaceutical composition for the suppression of thromboembolism after stent placement, comprising edoxaban or a pharmaceutically acceptable salt thereof, or a hydrate of the compound or the salt, a method for suppressing thromboembolism after stent placement using edoxaban or a pharmaceutically acceptable salt thereof, or a hydrate of the compound or the salt, and combined use or a combination drug of edoxaban or a pharmaceutically acceptable salt thereof, or a hydrate of the compound or the salt with an antiplatelet drug for the suppression of thromboembolism after stent placement.

### Background Art

Edoxaban tosylate hydrate competitively and selectively inhibits activated blood coagulation factor X (hereinafter, referred to as "FXa") in mammals such as humans.
Pharmaceutical compositions containing edoxaban tosylate hydrate have indications and usage, such as for reducting the risks of stroke and systemic embolism in nonvalvular atrial fibrillation patients, and for the treatment of deep vein thrombosis and pulmonary embolism (see e.g., Non-Patent Literatures 1 and 2).
Antiplatelet drugs such as aspirin and clopidogrel are used for suppressing thromboembolism after stent placement (see e.g., Non-Patent Literature 3).

### Citation List

### Non-Patent Literature

Non-Patent Literature 1: Blood (ASH Annual Meeting Abstracts) 2012 120: Abstract 4697
Non-Patent Literature 2: SAVAYSA Label
Non-Patent Literature 3: Clinical Therapeutics, Vol. 32, No. 14, 2010, p. 2265-2281

### Summary of Invention

### Technical Problem

An object of the present invention is to provide a method for suppressing thromboembolism after stent placement using an FXa inhibitor such as edoxaban.

### Solution to Problem

The present invention provides:

(1) a pharmaceutical composition for the suppression of thromboembolism after stent placement, comprising edoxaban or a pharmaceutically acceptable salt thereof, or a hydrate of the compound or the salt;
(2) the pharmaceutical composition according to (1), further comprising an antiplatelet drug;
(3) the pharmaceutical composition according to (2), wherein the antiplatelet drug is at least one compound selected from the group consisting of aspirin, ticlopidine, clopidogrel, prasugrel, elinogrel, ticagrelor, cangrelor, cilostazol, abciximab, eptifibatide, tirofiban, vorapaxar and dipyridamole, or a pharmaceutically acceptable salt thereof;
(4) use of edoxaban or a pharmaceutically acceptable salt thereof, or a hydrate of the compound or the salt for producing a pharmaceutical composition for the suppression of thromboembolism after stent placement;
(5) the use according to (4), wherein an antiplatelet drug is further used;
(6) the use according to (5), wherein the antiplatelet drug is at least one compound selected from the group consisting of aspirin, ticlopidine, clopidogrel, prasugrel, elinogrel, ticagrelor, cangrelor, cilostazol, abciximab, eptifibatide, tirofiban, vorapaxar and dipyridamole, or a pharmaceutically acceptable salt thereof;
(7) a method for suppressing thromboembolism after stent placement, comprising administering edoxaban or a pharmaceutically acceptable salt thereof, or a hydrate of the compound or the salt to a mammal;
(8) the method according to (7), comprising administering an antiplatelet drug to the mammal at the same time as or separately from the edoxaban or the pharmaceutically acceptable salt thereof, or the hydrate of the compound

or the salt;

(9) the method according to (7) or (8), wherein the mammal is a human;

(10) the method according to (8) or (9), wherein the antiplatelet drug is at least one compound selected from the group consisting of aspirin, ticlopidine, clopidogrel, prasugrel, elinogrel, ticagrelor, cangrelor, cilostazol, abciximab, eptifibatide, tirofiban, vorapaxar and dipyridamole, or a pharmaceutically acceptable salt thereof;

(11) a suppressive formulation for thromboembolism after stent placement, comprising edoxaban or a pharmaceutically acceptable salt thereof, or a hydrate of the compound or the salt in combination with an antiplatelet drug;

(12) the suppressive formulation according to (11), wherein the antiplatelet drug is at least one compound selected from the group consisting of aspirin, ticlopidine, clopidogrel, prasugrel, elinogrel, ticagrelor, cangrelor, cilostazol, abciximab, eptifibatide, tirofiban, vorapaxar and dipyridamole, or a pharmaceutically acceptable salt thereof;

(13) a method for suppressing thromboembolism after stent placement, comprising administering edoxaban or a pharmaceutically acceptable salt thereof, or a hydrate of the compound or the salt to a mammal given an antiplatelet drug;

(14) the method according to (13), wherein the mammal is a human;

(15) the method according to (13) or (14), wherein the antiplatelet drug is at least one compound selected from the group consisting of aspirin, ticlopidine, clopidogrel, prasugrel, elinogrel, ticagrelor, cangrelor, cilostazol, abciximab, eptifibatide, tirofiban, vorapaxar and dipyridamole, or a pharmaceutically acceptable salt thereof;

(16) a method for suppressing thromboembolism after stent placement, comprising administering an antiplatelet drug to a mammal given edoxaban or a pharmaceutically acceptable salt thereof, or a hydrate of the compound or the salt;

(17) the method according to (16), wherein the mammal is a human;

(18) the method according to (16) or (17), wherein the antiplatelet drug is at least one compound selected from the group consisting of aspirin, ticlopidine, clopidogrel, prasugrel, elinogrel, ticagrelor, cangrelor, cilostazol, abciximab, eptifibatide, tirofiban, vorapaxar and dipyridamole, or a pharmaceutically acceptable salt thereof;

(19) a method for suppressing thromboembolism after stent placement, comprising administering an formulation containing both edoxaban or a pharmaceutically acceptable salt thereof, or a hydrate of the compound or the salt and an antiplatelet drug to a mammal;

(20) the method according to (19), wherein the mammal is a human; and

(21) the method according to (19) or (20), wherein the antiplatelet drug is at least one compound selected from the group consisting of aspirin, ticlopidine, clopidogrel, prasugrel, elinogrel, ticagrelor, cangrelor, cilostazol, abciximab, eptifibatide, tirofiban, vorapaxar and dipyridamole, or a pharmaceutically acceptable salt thereof.

## Advantageous Effects of Invention

The present invention produces an effect in that a novel pharmaceutical composition that can suppress thromboembolism after stent placement can be provided. The present invention further produces an effect in that, even if the dose of an antiplatelet drug conventionally used for suppressing thromboembolism after stent placement is decreased, thromboembolism after stent placement can be sufficiently suppressed by further administering edoxaban or a pharmaceutically acceptable salt thereof, or a hydrate of the compound or the salt.

## Brief Description of Drawings

[Figure 1] Figure 1 is a diagram showing the antithrombotic effect and anti-FXa effect of edoxaban tosylate hydrate in rat models with stent thrombosis. A shows the amount of protein in thrombus. B shows FXa activity. In the diagram, the bars represent mean ± standard deviation (n = 8). The values within the parentheses represent the rate of inhibition (%). P values (Dunnett's test) compared with a control group are indicated by the number of *. *: $P < 0.05$, **: $P < 0.01$ and ***: $P < 0.001$.

[Figure 2] Figure 2 is a diagram showing the antithrombotic effect of aspirin in rat models with stent thrombosis. In the diagram, the bars represent mean ± standard deviation (n = 4 to 8). The values within the parentheses represent the rate of inhibition (%). P values (Dunnett's test) compared with a control group are indicated by the number of *. **: $P < 0.01$ and ***: $P < 0.001$.

[Figure 3] Figure 3 is a diagram showing the antithrombotic effect of clopidogrel sulfate in rat models with stent thrombosis. In the diagram, the bars represent mean ± standard deviation (n = 4 to 8). The values within the parentheses represent the rate of inhibition (%). P values (Dunnett's test) compared with a control group are indicated by the number of *. $P < 0.05$ and ***: $P < 0.001$.

[Figure 4] Figure 4 is a diagram showing the antithrombotic effect of ticagrelor sulfate in rat models with stent

thrombosis. In the diagram, the bars represent mean ± standard deviation (n = 8). The values within the parentheses represent the rate of inhibition (%). P values (Dunnett's test) compared with a control group are indicated by the number of *. P < 0.05, **: P < 0.01 and ***: P < 0.001.

[Figure 5] Figure 5 is a diagram showing the antithrombotic effect and anti-FXa effect of the combined use of aspirin and edoxaban tosylate hydrate in rat models with stent thrombosis. A shows the amount of protein in thrombus. B shows FXa activity. In the diagram, the bars represent mean ± standard deviation (n = 11 or 12). Edo denotes edoxaban tosylate hydrate, and ASA denotes aspirin. The values within the parentheses represent the rate of inhibition (%). In A, ** represents that a P value (Welch's test) compared with a control group was less than 0.01, and # represents that a P value (Student's t-test) compared with an aspirin-alone group was less than 0.05. In B, *** represents that a P value (Welch's test) compared with a control group was less than 0.001, and ### represents that a P value (Welch's test) compared with an aspirin-alone group was less than 0.001.

[Figure 6] Figure 6 is a diagram showing the antithrombotic effect and anti-FXa effect of the combined use of clopidogrel sulfate and edoxaban tosylate hydrate in rat models with stent thrombosis. A shows the amount of protein in thrombus. B shows FXa activity. In the diagram, the bars represent mean ± standard deviation (n = 10). Edo denotes edoxaban tosylate hydrate, and, Clopido denotes clopidogrel sulfate. The values within the parentheses represent the rate of inhibition (%). In A, *** represents that a P value (Student's t-test) compared with a control group was less than 0.001, and ### represents that a P value (Student's t-test) compared with a clopidogrel sulfate-alone group was less than 0.001. In B, *** represents that a P value (Welch's test) compared with a control group was less than 0.001, and ### represents that a P value (Welch's test) compared with a clopidogrel sulfate-alone group was less than 0.001.

Description of Embodiments

Examples of the "stent" include, but are not limited to, metal stents, resin stents, drug-eluting stents and bioabsorbable stents.

The site where the stent is placed is not particularly limited as long as the site is within a blood vessel. The site is preferably a carotid artery, a peripheral artery, an intracranial artery or a coronary artery.

In the present specification, the term "edoxaban" means N-(5-chloropyridin-2-yl)-N'-[(1S,2R,4S)-4-(dimethylcarbamoyl)-2-(5-methyl-4,5,6,7-tetrahydro[1,3]thiazolo[5,4-c]pyridine-2-carboxamido)cyclohexyl]oxamide represented by the following formula:

[Formula 1]

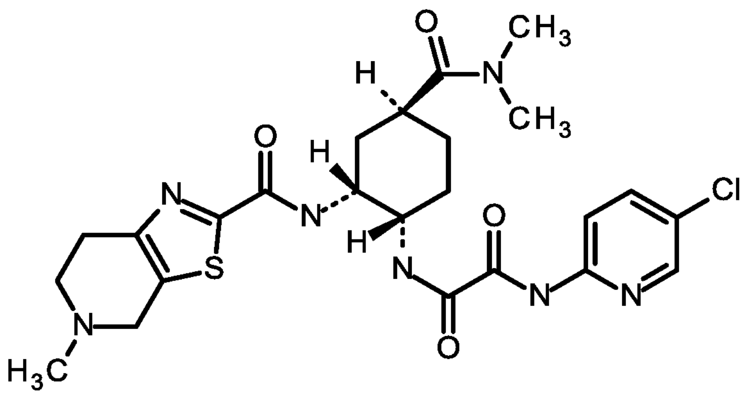

unless otherwise specified.

Examples of the salt of edoxaban include hydrochloride, sulfate, hydrobromide, citrate, hydroiodide, phosphate, nitrate, benzoate, methanesulfonate, benzenesulfonic acid, 2-hydroxyethanesulfonate, tosylate, acetate, propanoate, oxalate, malonate, succinate, glutarate, adipate, tartrate, maleate, fumarate, malate and mandelate. The salt of edoxaban is preferably hydrochloride, tartrate or tosylate, more preferably tosylate.

The edoxaban or the pharmaceutically acceptable salt thereof, or the hydrate of the compound or the salt is preferably edoxaban tosylate hydrate represented by the following formula:

[Formula 2]

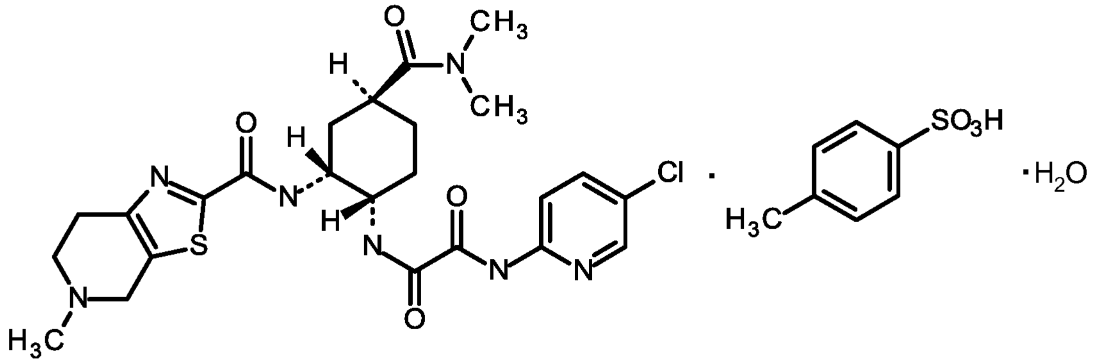

A commercially available product can be used as the edoxaban or the pharmaceutically acceptable salt thereof, or the hydrate of the compound or the salt.

Examples of the antiplatelet drug used in the present invention include aspirin, ticlopidine, clopidogrel, prasugrel, elinogrel, ticagrelor, cangrelor, cilostazol, abciximab, eptifibatide, tirofiban, vorapaxar and dipyridamole, and their salts. A commercially available product can be used as the antiplatelet drug.

In the present specification, the phrase "comprising edoxaban or a pharmaceutically acceptable salt thereof, or a hydrate of the compound or the salt in combination with an antiplatelet drug" refers to a form in which a formulation containing the edoxaban or the pharmaceutically acceptable salt thereof, or the hydrate of the compound or the salt is administered at the same time as or separately from a formulation containing the antiplatelet drug, or a form in which a formulation containing both of the edoxaban or the pharmaceutically acceptable salt thereof, or the hydrate of the compound or the salt and the antiplatelet drug (hereinafter, referred to as a "combination drug") is administered.

In the present specification, administration "at the same time" refers to administration at substantially the same time.

In the present specification, "separate" administration refers to separate administration at different times. For example, the antiplatelet drug is first administered, and subsequently, the edoxaban or the pharmaceutically acceptable salt thereof, or the hydrate of the compound or the salt is administered after a lapse of a predetermined time, or vice versa.

In the case of administering the pharmaceutical composition or the combination drug of the present invention to a mammal (e.g., a human, a horse, cattle or a pig, preferably a human), the administration can be systemic or local and oral or parenteral. A drug-eluting stent or balloon using edoxaban or a pharmaceutically acceptable salt thereof, or a hydrate of the compound or the salt as a drug is also included in the pharmaceutical composition of the present invention.

The pharmaceutical composition or the combination drug of the present invention can be prepared according to various formulation methods usually used by selecting an appropriate form according to administration method.

Examples of oral dosage forms of the pharmaceutical composition or the combination drug include tablets, powders, granules, capsules, suspensions, emulsions, syrups and elixirs. The pharmaceutical composition in such a form can be produced according to a routine method by selecting an additive usually used such as an excipient, a binder, a disintegrant, a lubricant, a swelling agent, a swelling aid, a coating agent, a plasticizer, a stabilizer, an antiseptic, an antioxidant, a colorant, a solubilizer, a suspending agent, an emulsifier, a sweetener, a preservative, a buffer, a diluent or a wetting agent according to need.

Examples of parenteral dosage forms of the pharmaceutical composition or the combination drug include injections, ointments, gels, creams, poultices, patches, aerosolized agents, inhalants, sprays, eye drops, nasal drops and suppositories. The pharmaceutical composition in such a form can be produced according to a routine method by selecting an additive usually used such as a stabilizer, an antiseptic, a solubilizer, a humectant, a preservative, an antioxidant, a flavor, a gelling agent, a neutralizing agent, a buffer, a tonicity agent, a surfactant, a colorant, a buffering agent, a thickener, a wetting agent, a filler, an absorption promoter, a suspending agent or a binder according to need.

When the pharmaceutical composition of the present invention is a drug-eluting stent or balloon, the pharmaceutical composition of the present invention can be produced according to a routine method by allowing the drug to be supported by a stent or a balloon.

The dose of the edoxaban or the pharmaceutically acceptable salt thereof, or the hydrate of the compound or the salt contained in the pharmaceutical composition of the present invention differs depending on symptoms, age, body mass, etc. For oral administration, the edoxaban or the pharmaceutically acceptable salt thereof, or the hydrate of the compound or the salt is administered once to several times a day at a dose of 1 to 200 mg, preferably 5 to 100 mg, in terms of the amount of edoxaban per dose in an adult, more preferably once a day at a dose of 15 to 60 mg in terms of

the amount of edoxaban. When the edoxaban or the pharmaceutically acceptable salt thereof, or the hydrate of the compound or the salt is used in combination with the antiplatelet drug, an appropriate decrease in its dose is also taken into consideration in light of the risk of bleeding.

The dose of the antiplatelet drug is set according to the package insert or the like of each agent. When the antiplatelet drug is used in combination with the edoxaban or the pharmaceutically acceptable salt thereof, or the hydrate of the compound or the salt, an appropriate decrease in its dose is also taken into consideration in light of the risk of bleeding. In the case of using, for example, aspirin, clopidogrel or prasugrel as the antiplatelet drug, each agent can be administered at doses of 75 to 325 mg/day, 50 to 75 mg/day and 3.75 to 10 mg/day, respectively.

The pharmaceutical composition or the combination drug thus obtained can be used for suppressing thromboembolism after stent placement. Even if the dose of an antiplatelet drug conventionally used for suppressing thromboembolism after stent placement is decreased, the pharmaceutical composition or the combination drug thus obtained can sufficiently suppress thromboembolism after stent placement by further administering edoxaban or a pharmaceutically acceptable salt thereof, or a hydrate of the compound or the salt. Therefore, the pharmaceutical composition or the combination drug can be used for suppressing thromboembolism after stent placement while reducing side effects.

Next, the present invention will be described in detail with reference to the Examples. However, the present invention is not intended to be limited by these in any way.

Examples

(Reagents)

The reagents used in the Examples are as follows.

0.5 w/v% Methyl Cellulose Solution 400 cP (hereinafter, referred to as a "0.5% MC solution") (Wako Pure Chemical Industries, Ltd.)
Thiopental sodium (Mitsubishi Tanabe Pharma Corporation)
Protein Assay CBB Solution, NaOH and 10 × Tris Buffered Saline (10 × TBS, pH 7.4) (Nacalai Tesque Inc.)
Bovine serum albumin (BSA) (Sigma Aldrich Co. LLC.)
Sodium citrate tribasic dihydrate (Sigma Aldrich Co. LLC.)
Albumin Standard (Bovine albumin, Thermo Fisher Scientific Inc.)
Human FXa (Enzyme Research Laboratories Ltd.)
S-2222 (substrate of FXa) (Chromogenix Instrumentation Laboratory S.p.A.)
Saline and distilled water (Otsuka Pharmaceutical Factory, Inc.)
$Na_2CO_3$ (Kishida Chemical Co., Ltd.)

(Material)

The stent used in the Examples is as follows.

Stent (material: stainless 316 L, outer diameter: 2.0 mm, length: 16 mm) (Kawasumi Laboratories, Inc.)

(Test substance)

The test substances used were edoxaban tosylate hydrate (hereinafter, abbreviated to "edoxaban"), aspirin, clopidogrel sulfate (hereinafter, abbreviated to "clopidogrel") and ticagrelor sulfate (hereinafter, abbreviated to "ticagrelor").

(Preparation of drug solution)

Each test substance was dissolved or suspended in a 0.5% MC solution to prepare a solution or a suspension for administration.

(Preparation of reagent solution)

2 × TBS containing 0.2% BSA: Before use, 10 × TBS was diluted with distilled water, and BSA (final concentration: 0.2%) was added thereto.

FXa: An FXa solution was prepared at 40 IU/mL with 2 × TBS containing 0.2% BSA and stored at -80°C. Before use, the frozen product was thawed and diluted into 0.032 IU/mL with the same solvent as above.

S-2222: An S-2222 solution was prepared at 3 mM with distilled water and stored at -20°C. Before use, the frozen product was thawed and diluted to 0.75 mM with distilled water.

(Animal)

The animals used in the Examples are as follows.

Species: Rat
Strain and sex: Slc:SD, male
Distributor: Japan SLC, Inc.
Age when used: 10 weeks old
Acclimatization period: 1 week or longer

(Rearing environment)

The acclimatization was carried out in the following environment.

Set temperature: 23°C
Humidity: 55 ± 10%
Illumination: 12 hr/day (7:00 to 19:00)
The number of rats reared: 3 individuals per cage
Feed and water: Each rat freely took solid feed FR-2 (Funabashi Farm Co., Ltd.) and tap water.

(Production of model)

Each rat was anesthetized with thiopental sodium (100 mg/mL/kg, i.p.) and placed on a warming mat. 18 minutes after the start of the anesthesia, polyethylene tubes (approximately 34 cm) containing four stents were inserted into the right and left carotid arteries and jugular veins to produce arteriovenous shunts (hereinafter, referred to as "AV shunts"). Blood flow was started in the shunts. 30 minutes after the start of the blood flow, the stents with thrombus attached thereto were taken out, and the amount of protein in the thrombus was measured.

(Example 1) Antithrombotic effects of edoxaban, aspirin, clopidogrel and ticagrelor

Edoxaban (0.3, 1 or 3 mg/10 mL/kg) 30 minutes before the AV shunting or aspirin (1, 3, 5, 10, 25, 50 or 100 mg/15 mL/kg), clopidogrel (1, 3, 10 or 30 mg/10 mL/kg) or ticagrelor (0.3, 1 or 3 mg/10 mL/kg) two hours before the AV shunting was orally administered to each rat fasted overnight. A 0.5% MC solution (10 mL/kg or 15 mL/kg) was orally administered to a control group. 12 minutes after the administration of edoxaban or 1 hour and 42 minutes after the administration of aspirin, clopidogrel or ticagrelor, the rat was anesthetized with thiopental sodium, and thrombus models were produced by the method described in the preceding paragraph "Production of model". The collected thrombus-attached stents were stored at -20°C until protein quantification. For the evaluation of edoxaban, 0.36 mL of blood was collected, immediately before the collection of the thrombus-attached stents, from the jugular vein using a syringe packed with a 1/10 volume (0.04 mL) of a 3.13% aqueous sodium citrate tribasic dihydrate solution. The collected blood was centrifuged (universal refrigerated centrifuge 5930, Kubota Corporation) at 4°C at 1,500 × g for 10 minutes to separate plasma. Then, the anti-FXa activity in the plasma was measured.

(Example 2) Antithrombotic effect of combined use of edoxaban and aspirin or clopidogrel

Aspirin (5 mg/15 ml/kg) or clopidogrel (3 mg/10 ml/kg) was orally administered to each rat fasted overnight. 1.5 hours thereafter, edoxaban (0.5 mg/10 mL/kg) was orally administered thereto. A 0.5% MC solution (10 mL/kg or 15 mL/kg) was orally administered to a control group. 12 minutes after the administration of edoxaban, the rat was anesthetized with thiopental sodium, and thrombus models were produced by the method described in the preceding paragraph "Production of model". The antithrombotic effect was evaluated in the same way as in Example 1.

(Measurement of parameter)

Amount of protein in thrombus: The thrombus-attached stents were placed in 0.9 mL of a 0.1 N NaOH solution containing 2% $Na_2CO_3$ and boiled for approximately 30 minutes to solubilize protein. The amount of protein in the thrombus lysate was determined by the Bradford method (Anal Biochem. 1976; 72: 248-254).

Anti-FXa activity in plasma: The rate of inhibition against S-2222 degradation reaction by human FXa was calculated for plasma samples. 5 μL of the plasma and 5 μL of purified water were added to each well of a 96-well plate. Subsequently, 50 μL of 0.032 IU/mL FXa diluted with 2 × TBS containing 0.2% BSA was added to each well. The reaction was started by the addition of 40 μL/well of 0.75 mM S-2222. The absorbance at 405 nm was measured at room temperature for 10 minutes using an absorption spectrometer SPECTRAmax 190 (Molecular Devices, LLC). The anti-FXa activity (rate of inhibition) in the plasma was calculated from the calculated reaction rate (FXa activity, ΔO.D./min) according to the following expression:

Anti-FXa activity in the plasma (%) = (1 - ΔO.D./min in the plasma of each individual rat given the test substance / Average ΔO.D./min in the plasma of the control group rats) × 100

(Statistical process)

The results were indicated by mean ± standard deviation. The analysis was carried out using SAS System Release 8.2 and Microsoft Excel 2010. The analysis was conducted by the two-tailed test for all of the samples with a significance level of less than 5%.

Example 1 (Antithrombotic effect of test substance): As for the amount of protein in the thrombus and FXa activity in the plasma, the control group and each dose group of each test substance were compared by the Dunnett's test, and the dose responsiveness was evaluated by the Spearman's correlation coefficient test.

Example 2 (Antithrombotic effect of combined use of edoxaban and antiplatelet drug): As for the amount of protein in the thrombus and FXa activity in the plasma, the homoscedasticity between the groups was confirmed by the F test, and the groups were then compared by the Student's t-test or the Welch's test.

(Results)

(Example 1)

The rat stent thrombosis models were experimented 5 times. In the experiments, the average amount of protein in the thrombus of the control group was 1025 to 2953 μg.

Edoxaban administered at 0.3, 1 and 3 mg/kg suppressed 37% (P = 0.407), 70% (P = 0.042) and 86% (P = 0.011), respectively, of the thrombus formation (Figure 1 A). The antithrombotic effect of edoxaban was found to be dose-dependent (P < 0.001). The dose-dependent (P < 0.001) and significant anti-FXa activity was confirmed in the plasma after the oral administration of edoxaban (Figure 1 B).

Aspirin administered at 5 and 10 mg/kg exhibited 40% and 47% rates, respectively, of suppression of thrombus formation, but produced no significant effect (Figure 2 A). Aspirin administered at 10, 25, 50 and 100 mg/kg suppressed 63% (P = 0.001), 64% (P < 0.001), 70% (P < 0.001) and 83% (P < 0.001), respectively, of the thrombus formation (Figure 2B). The antithrombotic effect of aspirin was found to be dose-dependent (P < 0.001).

Clopidogrel administered at 1, 3, 10 and 30 mg/kg suppressed 40% (P = 0.075), 47% (P = 0.024), 84% (P < 0.001) and 94% (P < 0.001), respectively, of the thrombus formation (Figure 3). The antithrombotic effect of clopidogrel was found to be dose-dependent (P < 0.001).

Ticagrelor administered at 0.3, 1 and 3 mg/kg suppressed 67% (P = 0.014), 74% (P = 0.006) and 89% (P < 0.001), respectively, of the thrombus formation (Figure 4). The antithrombotic effect of ticagrelor was found to be dose-dependent (P < 0.001).

(Example 2)

The effect of the combined use of edoxaban (0.5 mg/kg) and aspirin (5 mg/kg) was evaluated in the rat stent thrombosis models. The amount of protein in the thrombus of the control group was 1692 ± 304 μg (Figure 5 A). Edoxaban and aspirin both exhibited 42% rate of suppression of thrombus formation when each used alone (P value: 0.060 and 0.056, respectively). The combined use group of edoxaban and aspirin exhibited a significant suppressive effect of 65% (P = 0.004 vs. control) and significantly decreased the amount of thrombus as compared with the aspirin-alone group (P = 0.049 vs. aspirin). This combined use was not significant with respect to the edoxaban-alone group (P

= 0.054 vs. edoxaban). The anti-FXa activity in the plasma was equivalent between the edoxaban-alone group and the combined use group of edoxaban and aspirin (Figure 5 B).

Next, the effect of the combined use of edoxaban (0.5 mg/kg) and clopidogrel (3 mg/kg) was evaluated. The amount of protein in the thrombus of the control group was 1209 $\pm$ 151 $\mu$g (Figure 6 A). Edoxaban and clopidogrel exhibited 25% (P = 0.340) and 21% (P = 0.149) rates, respectively, of suppression of thrombus formation when each used alone. The combined use group of edoxaban and clopidogrel exhibited a significant suppressive effect of 61% (P < 0.001 vs. control) and significantly decreased the amount of thrombus as compared with the clopidogrel-alone group (P < 0.001 vs. clopidogrel). This combined use was not significant with respect to the edoxaban-alone group (P = 0.166 vs. edoxaban). The anti-FXa activity in the plasma was equivalent between the edoxaban-alone group and the combined use group of edoxaban and clopidogrel (Figure 6 B).

As seen from these results, the FXa inhibitor edoxaban exhibited a dose-dependent and significant antithrombotic effect, as with the antiplatelet drugs aspirin, clopidogrel and ticagrelor, in the rat stent thrombosis models. These models also revealed that edoxaban at a low dose plus an antiplatelet drug (aspirin or clopidogrel) at a low dose significantly decreases the amount of thrombus, as compared with the administration of the antiplatelet drug alone.

## Claims

1. A pharmaceutical composition for the suppression of thromboembolism after stent placement, comprising edoxaban or a pharmaceutically acceptable salt thereof, or a hydrate of the compound or the salt.

2. The pharmaceutical composition according to claim 1, further comprising an antiplatelet drug.

3. The pharmaceutical composition according to claim 2, wherein the antiplatelet drug is at least one compound selected from the group consisting of aspirin, ticlopidine, clopidogrel, prasugrel, elinogrel, ticagrelor, cangrelor, cilostazol, abciximab, eptifibatide, tirofiban, vorapaxar and dipyridamole, or a pharmaceutically acceptable salt thereof.

4. Use of edoxaban or a pharmaceutically acceptable salt thereof, or a hydrate of the compound or the salt for producing a pharmaceutical composition for the suppression of thromboembolism after stent placement.

5. The use according to claim 4, wherein an antiplatelet drug is further used.

6. The use according to claim 5, wherein the antiplatelet drug is at least one compound selected from the group consisting of aspirin, ticlopidine, clopidogrel, prasugrel, elinogrel, ticagrelor, cangrelor, cilostazol, abciximab, eptifibatide, tirofiban, vorapaxar and dipyridamole, or a pharmaceutically acceptable salt thereof.

7. A method for suppressing thromboembolism after stent placement, comprising administering edoxaban or a pharmaceutically acceptable salt thereof, or a hydrate of the compound or the salt to a mammal.

8. The method according to claim 7, comprising administering an antiplatelet drug to the mammal at the same time as or separately from the edoxaban or the pharmaceutically acceptable salt thereof, or the hydrate of the compound or the salt.

9. The method according to claim 7 or 8, wherein the mammal is a human.

10. A suppressive formulation for thromboembolism after stent placement, comprising edoxaban or a pharmaceutically acceptable salt thereof, or a hydrate of the compound or the salt in combination with an antiplatelet drug.

11. The suppressive formulation according to claim 10, wherein the antiplatelet drug is at least one compound selected from the group consisting of aspirin, ticlopidine, clopidogrel, prasugrel, elinogrel, ticagrelor, cangrelor, cilostazol, abciximab, eptifibatide, tirofiban, vorapaxar and dipyridamole, or a pharmaceutically acceptable salt thereof.

[Figure 1]

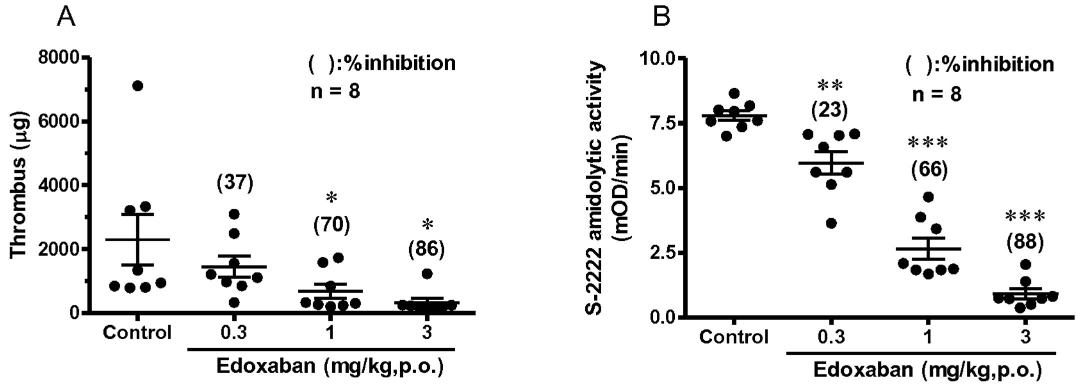


[Figure 2]

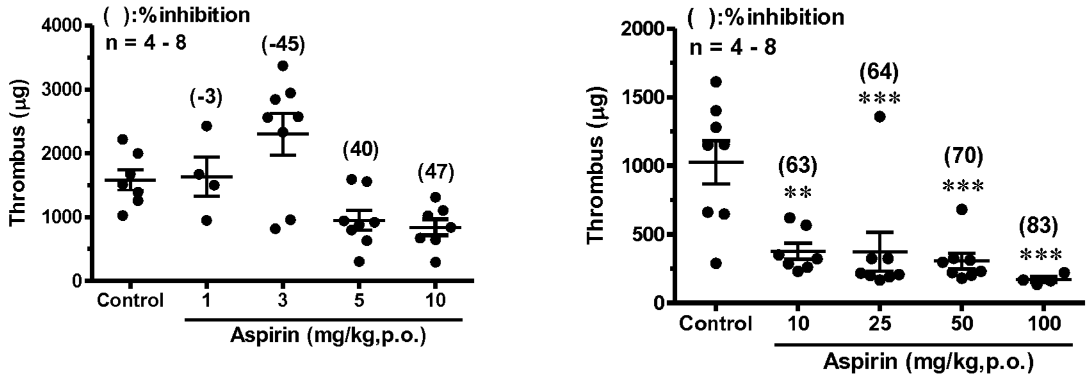


[Figure 3]

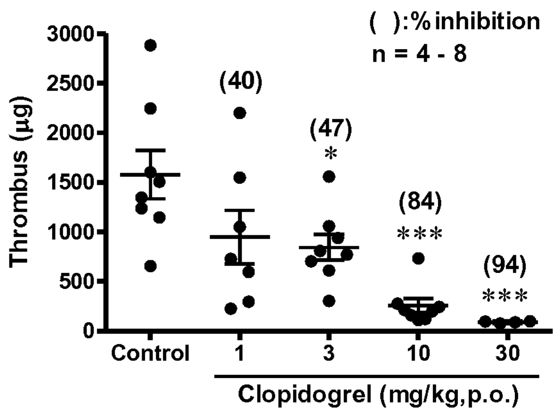

[Figure 4]

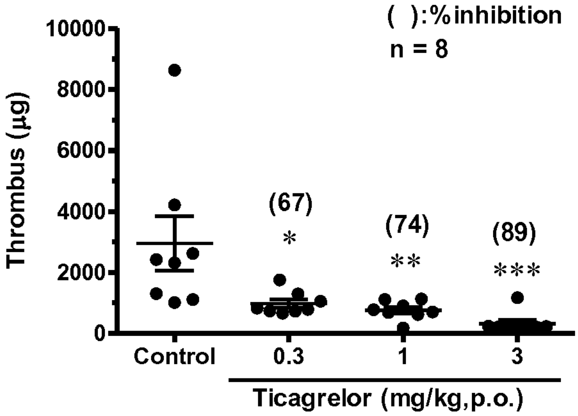


[Figure 5]

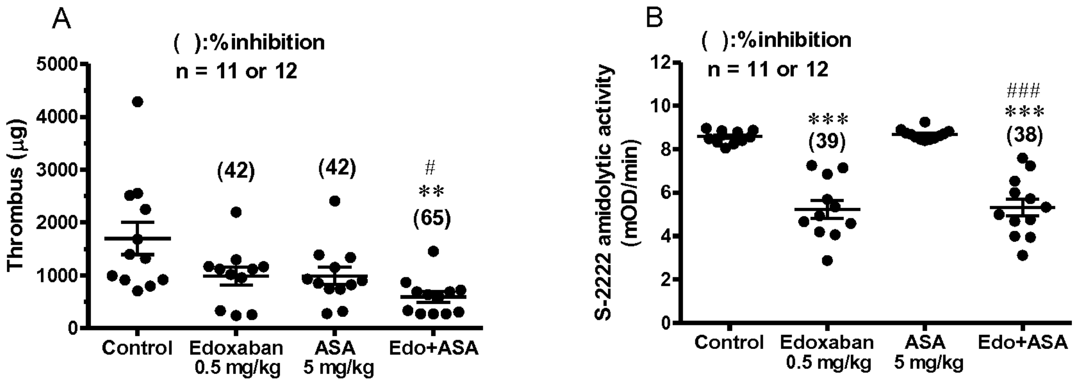


[Figure 6]

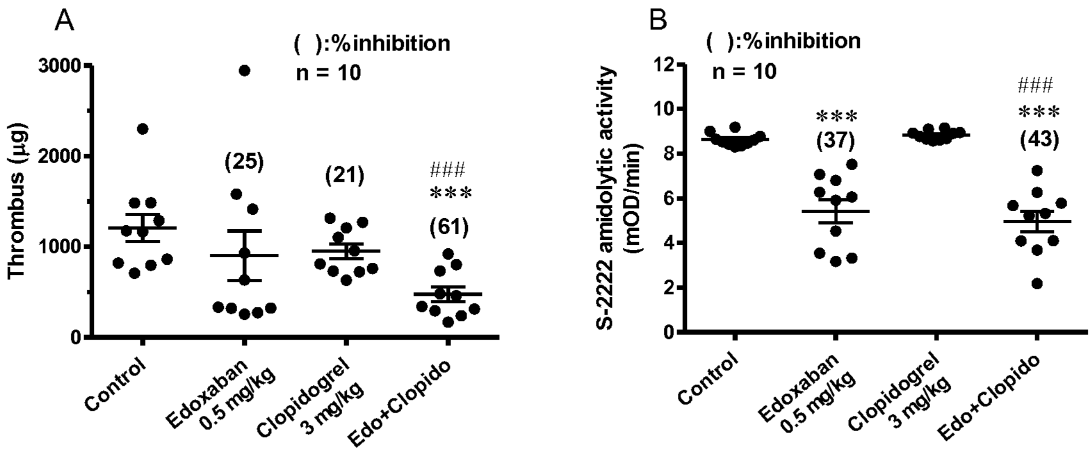

**INTERNATIONAL SEARCH REPORT**

International application No.
PCT/JP2015/074816

A. CLASSIFICATION OF SUBJECT MATTER
*A61K31/437*(2006.01)i, *A61K31/4365*(2006.01)i, *A61K31/4465*(2006.01)i, *A61K31/4709*(2006.01)i, *A61K31/519*(2006.01)i, *A61K31/616*(2006.01)i, *A61K45/00*(2006.01)i, *A61P7/02*(2006.01)i

According to International Patent Classification (IPC) or to both national classification and IPC

B. FIELDS SEARCHED

Minimum documentation searched (classification system followed by classification symbols)
A61K31/437, A61K31/4365, A61K31/4465, A61K31/4709, A61K31/519, A61K31/616, A61K45/00, A61P7/02

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2015 |
| Kokai Jitsuyo Shinan Koho | 1971-2015 | Toroku Jitsuyo Shinan Koho | 1994-2015 |

Electronic data base consulted during the international search (name of data base and, where practicable, search terms used)
JSTPlus/JMEDPlus/JST7580(JDreamIII), CAplus/REGISTRY/MEDLINE/EMBASE/BIOSIS(STN), PubMed

C. DOCUMENTS CONSIDERED TO BE RELEVANT

| Category* | Citation of document, with indication, where appropriate, of the relevant passages | Relevant to claim No. |
|---|---|---|
| Y | KARJALAINEN PP, et al., Safety and efficacy of combined antiplatelet-warfarin therapy after coronary stenting, European Heart Journal, 2007, Vol.28, p.726-732 Abstract, Introduction, Table4, Figure 2 | 1-11 |
| Y | Akimasa SUDO et al., "Kyusei Kanshokogun -Saishin no Chiken to Kangaekata-", Heart View, 09 February 2014 (09.02.2014), vol.18, no.2, pages (181)61 to (187)67, page 61, line 1 to page 62, left column, line 12 | 1-11 |
| Y | Toshikazu JINNAI et al., "DES ni Taisuru Stent Kessensho to sono Taisaku", Shinzo, 2009, vol.41, no.3, pages 268 to 272, page 268, left column, line 1 to page 269, right column, line 7 | 1-11 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 26 October 2015 (26.10.15) | 10 November 2015 (10.11.15) |

| Name and mailing address of the ISA/ | Authorized officer |
|---|---|
| Japan Patent Office<br>3-4-3,Kasumigaseki,Chiyoda-ku,<br>Tokyo 100-8915,Japan | <br>Telephone No. |

Form PCT/ISA/210 (second sheet) (July 2009)

**INTERNATIONAL SEARCH REPORT**

International application No. PCT/JP2015/074816

C (Continuation). DOCUMENTS CONSIDERED TO BE RELEVANT

| Category* | Citation of document, with indication, where appropriate, of the relevant passages | Relevant to claim No. |
|---|---|---|
| Y | Yoshiyuki MORISHIMA et al., "Research and development strategy of antithrombotic agents: pharmacology of the oral factor Xa-inhibitor edoxaban", Folia Pharmacol.Jpn., 2010, vol.136, pages 83 to 87, abstract, page 85, left column, 11th line from the bottom to right column, line 10 | 1-11 |
| Y | DEWILDE WJM, et al., Use of clopidogrel with or without aspirin in patients taking oral anticoagulant therapy and undergoing percutaneous coronary intervention: an open-label, randomised, controlled trial., Lancet, 2013, Vol.381, p.1107-1115 Summary, Introduction, page 1107, right column, lines 3 to 7 from the bottom, page 1111, left column, 2nd paragraph, page 1113, left column, 2nd paragraph, Table 3-5, Figure 3 | 1-11 |
| A | KRASNER A, et al., Antithrombotic therapy for patients with nonvalvular atrial fibrillation undergoing percutaneous coronary intervention: A review, Curr Cardiol Rep, 2013, Vol.15, No.378, p.1-8 Abstract, Introduction | 1-11 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *Clinical Therapeutics,* 2010, vol. 32 (14), 2265-2281
- *Anal Biochem.,* 1976, vol. 72, 248-254